(19) Europäisches Patentamt
European Patent Office
Office européen des brevets

(11) **EP 2 701 538 B1**

(12) **EUROPÄISCHE PATENTSCHRIFT**

(45) Veröffentlichungstag und Bekanntmachung des Hinweises auf die Patenterteilung:
**18.02.2015  Patentblatt 2015/08**

(21) Anmeldenummer: **12720827.0**

(22) Anmeldetag: **23.04.2012**

(51) Int Cl.:
*A23L 1/318* *(2006.01)*     *A23L 1/314* *(2006.01)*
*A23B 4/22* *(2006.01)*      *A23L 3/3571* *(2006.01)*
*C12N 9/62* *(2006.01)*      *C12R 1/645* *(2006.01)*
*C12R 1/80* *(2006.01)*

(86) Internationale Anmeldenummer:
**PCT/EP2012/057417**

(87) Internationale Veröffentlichungsnummer:
**WO 2012/146564 (01.11.2012 Gazette 2012/44)**

(54) **VERFAHREN ZUR VEREDELUNG VON FRISCHFLEISCH**

METHOD FOR REFINING FRESH MEAT

PROCÉDÉ D'ENRICHISSEMENT DE VIANDE FRAÎCHE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.04.2011  EP 11163616**
**19.06.2011  PCT/EP2011/060175**
**14.12.2011  EP 11193630**

(43) Veröffentlichungstag der Anmeldung:
**05.03.2014  Patentblatt 2014/10**

(73) Patentinhaber:
• **Tessaro, Marco**
**8003 Zürich (CH)**
• **Oechslin, Lucas**
**8005 Zürich (CH)**

(72) Erfinder:
• **Tessaro, Marco**
**8003 Zürich (CH)**
• **Oechslin, Lucas**
**8005 Zürich (CH)**

(74) Vertreter: **Dr. Graf & Partner AG**
**Intellectual Property**
**Herrenacker 15**
**Postfach 518**
**8200 Schaffhausen (CH)**

(56) Entgegenhaltungen:
**EP-A1- 0 724 843     EP-A1- 0 724 844**
**GB-A- 875 339       US-A- 2 816 836**
**US-A- 2 852 391      US-A- 3 056 679**
**US-A- 3 128 191**

• **MARTIN ALBERTO; ASENSIO MIGUEL A; BERMUDEZ MARIA E; CORDOBA MARIA G; ARANDA EMILIO; CORDOBA JUAN J: "Proteolytic activity of Penicillium chrysogenum and Debaryomyces hansenii during controlled ripening of pork loins", MEAT SCIENCE, Bd. 62, 1. September 2002 (2002-09-01), Seiten 129-137, XP002673694,**

• **OCKERMAN H W; CESPEDES-SANCHEZ F J; ORTEGA-MARISCAL M A; MARTIN-SERRANO A M; TORRES-MUNOZ M C; LEON-CRESPO F: "Influence of temperature on proteolytic activity of indigenous Spanish molds in meat products", JOURNAL OF MUSCLE FOODS, Bd. 12, 11. April 2001 (2001-04-11), Seiten 263-273, XP002673695,**

• **Benjamin Cordes: "Ein ausgereifter Genuss", , 20. August 2009 (2009-08-20), Seiten 1-2, XP002673722, Gefunden im Internet: URL:http://www.abendblatt.de/kultur-live/e ssen-trinken/article1147613/Ein-ausgereift er-Genuss.html [gefunden am 2012-04-11]**

## Beschreibung

Die Erfindung betrifft ein Verfahren zur Veredelung von Frischfleisch gemäss dem Oberbegriff von Anspruch 1.

## Stand der Technik

Nach der Schlachtung wird Frischfleisch einer so genannten Fleischreifung unterzogen. Die während der Fleischreifung ablaufenden Prozesse beeinflussen insbesondere die Zartheit, das Aroma und die Saftigkeit des Fleisches, weshalb diese Prozesse für die letztendlich erzielte Fleischqualität von entscheidender Bedeutung sind.

Die Fleischreifung ist ein Vorgang, der sich innerhalb der Muskelfasern des Fleisches abspielt. Die Fleischreifung beginnt bereits nach dem Schlachten während des Abkühlens des noch warmen Fleisches. Die Fleischreifung verläuft in zwei Phasen:

In der ersten Phase der Fleischreifung geht die zunächst weiche und schlaffe Muskulatur in die Totenstarre (Rigor mortis) über. Dies geschieht, weil mit der Schlachtung die Blutversorgung und damit die Sauerstoffversorgung der Muskeln unterbrochen wird. Der Stoffwechsel verläuft nun unter anaeroben Bedingungen. Dabei wird das in der Muskulatur befindliche Kohlenhydrat Glycogen zu Milchsäure (Lactat) abgebaut und Energie, das ATP, gewonnen. ATP wirkt im lebenden, zusammengezogenen (kontrahierten) Muskel als "Weichmacher". Sind die Glycogenvorräte verbraucht, kann kein ATP mehr gebildet werden, welches den Muskel nach der Kontraktion wieder weich und schlaff werden lässt. Die Muskulatur verharrt nun in einem angespannten, starren Zustand und ist gekennzeichnet durch eine maximale Zähigkeit und ein minimales Wasserbindevermögen.

In der zweiten Phase der Fleischreifung finden die für die letztendlich erzielte Fleischqualität wesentlichen Prozesse statt. Die Totenstarre löst sich beispielsweise beim Rind üblicherweise nach etwa 24 bis 30 Stunden. Die während der ersten Phase entstehende Milchsäure hat zur Folge, dass der pH-Wert von über 7 während des Schlachtens auf unter 5,8 absinkt. Freiwerdende, Eiweiss spaltende Enzyme, z.B. die Kathepsine und Calpaine, bewirken in der zweiten Phase eine Auflösung der Muskelfaserstrukturen (Myofibrillen), was die Zartheit des Fleisches zunehmend verbessert. Die dabei entstehenden freien Aminosäuren sind zudem wichtig für die Ausbildung des Aromas beziehungsweise für den Wohlgeschmack des Fleisches. Auch das Wasserbindevermögen nimmt während der Fleischreifung wieder zu und kann somit durch den Reifungsprozess beeinflusst werden. Die Fleischreifung beeinflusst somit die Zartheit, das Aroma und die Saftigkeit des Fleisches.

Es sind verschiedene Verfahren zur Fleischreifung bekannt.

Beim so genannten "Wet-aging" wird das Frischfleisch nach der Schlachtung vom Knochen gelöst und für die Fleischreifung in Luft dichten Beuteln vakuumverpackt. Durch dieses Verfahren kann kein Wasser aus dem Fleisch verdunsten, weshalb das Fleisch während der Reifung praktisch kein Gewicht verliert, was wirtschaftlich vorteilhaft ist. Das Fleisch wird nach einer bestimmten Lagerzeit der Vakuumverpackung entnommen und kann zum Verkauf angeboten werden.

Beim so genannten "Dry-aging"-Verfahren, in Deutsch auch als "knochenreifen" oder "am Knochen gereift" bezeichnet, wird das Frischfleisch nicht vom Knochen gelöst und nicht vakuumverpackt, was eine qualitativ hoch stehende Fleischreifung ermöglicht. Die Knochen, welche bei der Lagerung nicht entfernt wurden, bewirken, dass das Fleisch reicher an Aromen wird. Das Dry-aging weist den Nachteil auf, dass das Fleisch bedingt durch eine Kaltverdampfung des sich im Fleisch befindlichen Wassers an Gewicht verliert. Das Dry-aging weist den weiteren Nachteil auf, dass das Fleisch angetrocknete Oberflächen ausbildet, welche abgeschnitten werden müssen, was einen weiteren Gewichtsverlust zur Folge hat. Das Dry-aging weist gegenüber dem Wet-aging den Vorteil auf, dass das Fleisch ein verbessertes Aroma aufweist, beispielsweise wegen dem am Fleisch verbliebenen Knochen, und da die Aromastoffe im Fleisch bedingt durch den Wasserverlust konzentriert werden.

Bekannte Verfahren weisen somit bezüglich Gewichtsverlust, Aromastoffen und Zartheit des Fleisches Nachteile auf.

Das Dokument US 3 056 679 offenbart ein Verfahren zur beschleunigten Fleischreifung und zur Geschmacksverbesserung des Fleisches durch die Verwendung des Schimmelpilzes Thamnidium elegans, wobei das Fleisch zwischen 12 und 48 Stunden und bei 4.4 Grad C gereift wird.

Das Dokument US 3 128 191 offenbart ein Verfahren zur Geschmacksverbesserung des Fleisches durch die Verwendung des Schimmelpilzes Thamnidium elegans, wobei der Schimmelpilz dem noch lebenden Tier in einer Salinen Lösung injiziert wird, und wobei das Fleisch nach dem Schlachten des Tiers zwischen 1 und 10 Tagen bei Temperaturen zwischen 1,7 Grad C und 4.4 Grad C gereift wird.

Das Dokument GB 875 339 offenbart ein Verfahren zur beschleunigten Fleischreifung und zur Geschmacksverbesserung des Fleisches durch die Verwendung des Schimmelpilzes Thamnidium elegans, wobei das Fleisch während 48 Stunden gereift wird, wobei die Temperatur während den ersten 8 Stunden von 1,7 Grad C auf zwischen 15.6 Grad C und 24.9 Grad C erhöht wird, und wobei das Fleisch danach während 32 Stunden und einer Temperatur zwischen 18.3 Grad C und 24.9 Grad C gereift wird, und wobei das Fleisch danach während 8 Stunden und eine Temperatur zwischen 0 Grad C und 1.7 Grad C gekühlt wird.

Das Dokument US 2 816 836 offenbart ein Verfahren zur beschleunigten Fleischreifung und zur Geschmacksverbesserung des Fleisches durch die Verwendung des Schimmelpilzes Thamnidium elegans, wobei das Fleisch während 7 Tagen gereift wird, bei einer Temperatur von mehr als 7.2 Grad C und weniger als 15.6 Grad C.

Alle vier vorhin genannten Verfahren streben durch die Verwendung des Schimmelpilzes Thamnidium elegans eine Beschleunigung der Fleischreifung an. Derartige Verfahren werden auch als "fast aging"-Verfahren bezeichnet. Diese Verfahren weisen jedoch den Nachteil auf, dass der Geschmack und die Zartheit des Fleisches anspruchsvolle Fleischkenner nicht befriedigt.

**Darstellung der Erfindung**

Alle in der vorhergehenden Beschreibungseinleitung erwähnten Verfahren zur Fleischreifung bezwecken eine Veredelung von Frischfleisch, wobei die Merkmale des Fleisches, insbesondere die Fasrigkeit, erhalten bleiben. Das heisst diese Bearbeitungsverfahren reichen nicht aus die innere Muskelfaserstruktur des Fleisches zu verändern und so die Merkmale von Fleisch zu beseitigen. Die vorliegende Erfindung betrifft ein derartiges Verfahren zur Fleischreifung, bei dem die Merkmale des Fleisches, insbesondere die Fasrigkeit, erhalten bleiben.

Der Vollständigkeit halber sei erwähnt, dass Fleisch an sich in eine Vielzahl so genannter Fleischerzeugnisse weiter verarbeitet werden kann. Unter Fleischerzeugnisse werden Erzeugnisse verstanden, die aus der Verarbeitung von Fleisch oder Fleischerzeugnissen entstehen und die so beschaffen sind, dass bei einem Schnitt durch deren Kern die Schnittfläche die Feststellung erlaubt, dass die Merkmale von Fleisch, insbesondere die Fasrigkeit, nicht mehr vorhanden sind. Die Konservierung von Fleisch durch Pökelung stellt beispielsweise ein derartiges Fleischerzeugnis dar. Rohpökelerzeugnisse wie beispielsweise Schinken werden vorerst eingesalzen, unterliegen dann einer sogenannten Brennphase, in der in Lösung gegangenes Salz das Fleisch durchdringt, und werden dann in einem Klimaraum unter vorgegebenen Betriebsbedingungen gereift. Dabei werden die Fleischerzeugnisse derart verändert, dass bei einem Schnitt durch deren Kern die Schnittfläche die Feststellung erlaubt, dass die Merkmale von Fleisch nicht mehr vorhanden sind. Die vorliegende Erfindung betrifft keine derartigen Fleischerzeugnisse.

Aufgabe der Erfindung ist es ein verbessertes Verfahren zur Fleischreifung vorzuschlagen. Diese Aufgabe wird gelöst mit einem Verfahren aufweisend die Merkmale von Anspruch 1. Die Unteransprüche 2 bis 9 betreffen weitere, vorteilhafte Verfahrensschritte.

Die Aufgabe wird insbesondere gelöst mit einem Verfahren zur Veredelung von Frischfleisch ohne zusätzliche Salzzugabe, wobei zur oder während der Reifung des Fleisches zumindest ein Mikroorganismus aus der Gruppe: Penicillium nalgiovense, Penicillium chysogenum, Penicillium gladioli (P and B), Penicillium camemberti (M), zugegeben wird, und wobei das Fleisch in einem Temperaturbereich zwischen 0 bis 8 °C gelagert wird, und wobei das Fleisch bei einer relativen Luftfeuchtigkeit zwischen 50% und 98% gelagert wird, und wobei das Fleisch zwischen 21 bis 100 Tagen gelagert wird.

Das erfindungsgemässe Verfahren erfolgt ohne zusätzliche Salzzugabe, da, im Gegensatz zur Konservierung von Fleisch durch Pökelung, beim erfindungsgemässen Verfahren kein Salz beziehungsweise kein Pökelstoff zur Konservierung des Fleisches erforderlich ist. Das mit dem erfindungsgemässen Verfahren veredelte Fleisch weist nach Abschluss der Veredelung des Frischfleisches vorzugsweise einen Salzgehalt zwischen 0,1 - 2% auf, insbesondere zwischen 0,3 - 0,8%.

Beim erfindungsgemässen Verfahren werden zumindest einer der Parameter Temperatur, Luftfeuchtigkeit und Zeitdauer vorteilhafterweise derart beeinflusst, dass das Fleisch während der Reifung immer einen aw-Wert von grösser oder gleich 0.96 aufweist, vorzugsweise einen Wert zwischen 0.97 und 0.99, und insbesondere einen Wert von 0.98. Der aw-Wert, auch als Wasseraktivität bzw. in Englisch als "Activity of Water" bezeichnet, ist ein Maß für frei verfügbares Wasser in einem Material. Der $a_w$-Wert ist definiert als Quotient des Wasserdampfdrucks über einem Material ($p$) zu dem Wasserdampfdruck über reinem Wasser (po) bei einer bestimmten Temperatur:

$$a_w = \frac{p}{p_0}$$

Der $a_w$-Wert beeinflusst das Vorkommen beziehungsweise das Wachstum von Mikroorganismen, da diese je nach Mikroorganismus unterschiedliche Ansprüche an frei verfügbares Wasser haben. Bei Mangel an freiem Wasser werden die Wachstumsprozesse von gewissen Wasser liebenden Mikroorganismen verlangsamt, wobei empfindliche Mikroorganismen Organismen sogar abgetötet werden können. Xerophile Organismen hingegen wachsen bei sinkendem Wassergehalt besser.

Das erfindungsgemässe Verfahren weist den Vorteil auf, dass der $a_w$-Wert des damit erzeugten Fleisches nahe beim $a_w$-Wert von Frischfleisch liegt. Das gemäss dem erfindungsgemässen Verfahren hergestellte Fleisch kann daher wie ein Frischfleischprodukt verarbeitet und verzehrt werden, z.B. durch Braten, Schmoren oder Grillen. Das gemäss dem erfindungsgemässen Verfahren hergestellte Fleisch weist zudem die für Fleisch übliche Fasrigkeit auf.

Besonders vorteilhaft wird das Fleisch zwischen 28 bis 100 Tagen gelagert.

Das erfindungsgemässe Verfahren zur Veredelung von Frischfleisch weist den Vorteil auf, dass das Fleisch durch das Wirken des Mikroorganismus einerseits zarter wird, und dass das Fleisch durch das Wirken des Mikroorganismus einen Geschmack erhält. Es hat sich überraschenderweise gezeigt, dass eine Mehrzahl von Mikroorganismen geeignet sind das Frischfleisch während einer überaus langen Lagerzeit von zwischen 21 bis 100 Tagen zu reifen, wenn das Frischfleisch zudem in einem vorherbestimmten Temperaturbereich und einem vorherbestimmten Bereich relativer Luftfeuchtigkeit gelagert wird. Der durch das Wirken des Mikroorganismus verursachte Geschmack im Fleisch wird nach einer Lagerzeit von üblicherweise frühestens ab etwa 21 Tagen auf angenehme, als Qualitätsverbesserung empfundene Weise wahrgenommen, wobei der Geschmack sich mit zunehmender Lagerzeit intensiviert, sodass, abhängig vom verwendeten Mikroorganismus, der Temperatur und der Luftfeuchtigkeit zur optimalen Geschmacksentwicklung eine Gesamtlagerzeit von bis zu 50 Tagen oder bis zu 100 Tagen vorteilhaft ist.

Es hat sich gezeigt, dass die sich während der Fleischreifung entwickelnde Geschmacksnote des Fleisches abhängig ist vom verwendeten Mikroorganismus. Es erweist sich daher als besonders Vorteilhaft über eine Mehrzahl geeigneter Mikroorganismen zu verfügen, die zur erfindungsgemässen Fleischreifung geeignet sind. Das erfindungsgemässe Verfahren zur Veredelung von Frischfleisch ermöglicht es somit Fleisch mit unterschiedlichen Geschmacksnoten herzustellen, abhängig von dem jeweils verwendeten Mikroorganismus. So erzeugt der Schimmelpilz Thamnidium elegans beispielsweise einen nussigen Geschmack im Fleisch, wogegen beispielsweise der Schimmelpilz Penicillium nalgiovense einen eher nach Salami erinnernden Geschmack erzeugt. Die Vielfalt möglicher Geschmacksnoten des Fleisches und die Zartheit des Fleisches, welche mit dem erfindungsgemässen Verfahren herstellbar sind, begeistert anspruchsvolle Fleischkenner, was den zusätzliche Vorteil ergibt, dass das gemäss dem erfindungsgemässen Verfahren veredelte Frischfleisch wirtschaftlich vorteilhaft verkauft werden kann.

Im Verfahren wird als Mikroorganismus ein Schimmelpilz verwendet aus der Gruppe:

Penicillium nalgiovense, Penicillium chysogenum, Penicillium gladioli, Penicillium camemberti. Zudem ist als Mikroorganismus auch der Schimmelpilz Thamnidium elegans geeignet.

In einem besonders vorteilhaften Verfahren wird als Mikroorganismus ein Schimmelpilz verwendet aus der Gruppe: Penicillium nalgiovense, Penicillium chysogenum, Penicillium gladioli (P and B), Penicillium camemberti (M). Die Mikroorganismen dieser Gruppe sind besonderes gut geeignet für das erfindungsgemässe Verfahren:

- weil diese fähig sind auf dem Medium Fleisch zu wachsen,
- weil diese mit Proteasen und Lipasen arbeiten, welche zu einer Verbesserung der Zartheit und zu einer positiven Veränderungen im Geschmack führen,
- weil diese fähig sind bei einer Temperatur im Bereich zwischen 0 bis 8 °C effizient zu wachsen,
- weil diese teilweise fähig sind unerwünschte Mikroorganismen zu verdrängen,
- und weil diese keine oder nur schwache oder gut dokumentierte Mykotoxin-Bildner sind.

Die übrigen in Anspruch 1 beanspruchten Mikroorganismen weisen ebenfalls vorteilhafte Eigenschaften beim erfindungsgemässen Verfahren auf, insbesondere auch:

- weil diese durch Einsatz von Lipasen und anderen Enzymen eine Veränderung des Geschmacks bewirken,
- weil diese fähig sind bei einer Temperatur im Bereich zwischen 0 bis 8 °C zu wachsen,
- und weil diese teilweise fähig sind unerwünschte Mikroorganismen zu verdrängen.

Besonders vorteilhaft wird das Fleisch in einem Temperaturbereich zwischen 1 bis 7 °C, bei einer relativen Luftfeuchtigkeit zwischen 75% und 98%, und zwischen 21 bis 70 Tagen gelagert. Es hat sich insbesondere gezeigt, dass eine Lagerdauer von etwa 50 Tagen für gewisse Fleischstücke ideal ist. Die Lagerung bei einer relativen Luftfeuchtigkeit zwischen 75% bis 98% weist den Vorteil auf, dass das Fleisch nicht oder nur geringfügig austrocknet. Ein starkes Austrocknen hätte den Nachteil, dass sich das Gewicht des Fleisches reduziert.

Die während dem Reifen des Fleisches jeweils gewählte Temperatur und Luftfeuchtigkeit sowie die gewählte Lagerdauer ist insbesondere auch abhängig von dem zur Veredelung verwendeten Mikroorganismus, da je nach Mikroorganismus eine andere Kombination von Temperatur, Luftfeuchtigkeit und Lagerdauer optimal ist zur Entwicklung des Geschmacks und/oder der Zartheit des Fleisches. Es hat sich gezeigt, dass der vom Mikroorganismus bewirkte Geschmack üblicherweise erst ab einer Lagerzeit von etwa 21 Tagen wahrnehmbar ist, und dass die Intensität des Geschmacks danach mit zunehmender Lagerzeit ansteigt. In Kenntnis der erfindungsgemässen Lehre ist es daher sehr einfach für den jeweils verwendeten Mikroorganismus einen vorteilhaften Temperaturbereich bzw. einen vorteilhaften Luftfeuchtigkeitsbereiche und eine vorteilhafte Lagerdauer zu ermitteln, innerhalb welchem die Fleischreifung erfolgen sollte. Dazu genügt ein kleiner Versuch, bei welchem ein Fleisch mit dem entsprechenden Mikroorganismus mit einer bestimmten Temperatur, einer bestimmten Luftfeuchtigkeit und während einer Lagerdauer von zumindest 21 Tagen gereift wird und danach getestet wird. Somit lassen sich die zur Fleischveredelung vorteilhafte Temperatur, Luftfeuchtigkeit und Lagerdauer in Kenntnis der erfindungsgemässen Lehre auf einfache Weise ermitteln, falls die Werte für den entsprechend Mikroorganismus und das entsprechende Fleisch noch nicht bekannt sind. Es kann sich als vorteilhaft erweisen während der gesamten Fleischreifung dieselbe Temperatur und/oder Luftfeuchtigkeit beizubehalten. Es kann sich jedoch auch als vorteilhaft erweisen die Temperatur und/oder die Luftfeuchtigkeit in einem vorherbestimmten Bereich zu variieren.

In einem besonders vorteilhaften Verfahrensschritt wird die Temperatur nach einer Dauer von beispielsweise 28 bis 42 Tagen erhöht, wobei die Temperatur vorzugsweise um 1°C bis 2°C erhöht wird, um dadurch eine Beschleunigung der Fleischreifung zu erzielen. Vorteilhafterweise wird der letzte Zeitabschnitt der Fleischreifung bei erhöhter Temperatur durchgeführt, um dadurch eine schnellere Endreifung des Fleisches zu erzielen.

In einem weiteren, vorteilhaften Verfahrensschritt erfolgt während der Fleischreifung keine Temperaturveränderung, dafür wird Fleisch, das sich in unterschiedlichen Reifestadien befindet, in demselben Raum gelagert.

Besonders vorteilhaft werden Schimmelpilze mit einer Sporendichte von zumindest $10^9$ Sporen pro $cm^2$ auf die Oberfläche des Fleisches aufgetragen. Es kann sich als vorteilhaft erweisen eine höhere Sporendichte von zumindest $10^{12}$ Sporen pro $cm^2$ aufzutragen, vorzugsweise zwischen $10^{12}$ Sporen pro $cm^2$ bis $10^{15}$ Sporen pro $cm^2$. Der Schimmelpilz wird üblicherweise innerhalb von 3 Tagen nach der Schlachtung, vorzugsweise innerhalb von 2 Tagen nach der Schlachtung aufgetragen. Der Schimmelpilz kann notfalls noch bis zu 14 Tagen nach der Schlachtung aufgetragen werden. Um insbesondere ein Wachstum der Bakterien zu behindern und um einen genügend lange Einwirkzeit des Schimmelpilzes auf das Fleisch zu erzielen wird ein Aufbringen innerhalb von 2 bis 3 Tagen nach der Schlachtung bevorzugt.

Das erfindungsgemässe Verfahren weist den Vorteil auf, dass das behandelte Fleisch besonders zart und/oder saftig ist und eine in dieser Art bisher nicht gekannte, äusserst angenehme und abhängig von der Lagerzeit auch unterschiedlich intensive Geschmacksnote und abhängig vom verwendeten Organismus auch einen unterschiedlichen Geschmack aufweist. Das erfindungsgemässe Verfahren weist wie bereits eingangs beschrieben zudem den Vorteil auf, dass die Merkmale des Fleisches erhalten bleiben, weil das erfindungsgemässe Verfahren nicht in der Lage ist die innere Muskelfaserstruktur des Fleisches grundsätzlich zu verändern und so die Merkmale von Fleisch zu beseitigen. Dadurch kann das mit dem erfindungsgemässen Verfahren veredelte Fleisch in der Küche immer noch wie ein Frischfleischprodukt behandelt werden, zum Beispiel durch Braten, Schmoren oder Grillen, sodass das Fleisch vielseitig einsetzbar ist. Im Gegensatz zum Beispiel zu einem Fleischerzeugnis bei dem Fleisch mit einem Pökelstoff versetzt wurde, wie beispielsweise Schinken oder Rohschinken, die im Vergleich zu einem Frischfleischprodukt eine völlig unterschiedliche Konsistenz aufweisen. Nebst der Zerstörung der typischen Fleischmerkmale bzw. der typischen Fleischstruktur weisen Verfahren die Pökelstoffe verwenden auch den Nachteil auf, dass das Fleisch gesalzen wird. Das mit dem erfindungsgemässen Verfahren veredelte Fleisch weist somit den Vorteil auf, dass dieses einen niedrigen Salzgehalt aufweist und somit gesünder ist. Im Gegensatz zu gepökeltem Fleisch, das auf Grund des hohen Salzgehalten gesundheitliche Beschwerden wie Bluthochdruck zur Folge haben kann.

Bekannte Pökelverfahren verwenden Pökelstoffe beziehungsweise Salze zur Konservierung des Fleisches beziehungsweise zur Hemmung des Wachstums von Mikroorganismen. Nachteilig an diesen Verfahren ist die Tatsache, dass das Fleisch nicht natur belassen wird sondern gesalzen wird. Diese Verfahren weisen somit den Nachteil auf, dass als Konservierungsstoff Salz verwendet wird.

Das erfindungsgemässe Verfahren zur Veredelung von Frischfleisch läuft ohne Beigabe von Pökelstoffen ab beziehungsweise ohne zusätzliche Salzbeigabe ab, und verwendet dafür Mikroorganismen zur Konservierung des Fleisches beziehungsweise zur Hemmung des Wachstums schädlicher Mikroorganismen. Das erfindungsgemässe Verfahren ermöglicht es deshalb, dass die Merkmale des Fleisches erhalten bleiben.

Aus dem Stand der Technik ist es bekannt den Schimmelpilz Thamnidium elegans zu verwenden um die Fleischreifung zu beschleunigen, sodass das Fleisch innerhalb von maximal 10 Tagen gereift ist und zum Verkauf bereit steht.

Beim erfindungsgemässen Verfahren wurde überraschenderweise erkannt, dass die Verwendung der beanspruchten Mikroorganismen umfassend unter anderem auch Schimmelpilze bei einer bisher nicht gekannten, äusserst langen Lagerdauer von zwischen 21 bis 100 Tagen und vorzugsweise zwischen 28 bis 100 Tagen, im Fleisch eine besonders angenehme und falls erwünscht auch besonderes intensive Geschmacksnote entstehen lässt und eine ungewöhnliche Zartheit des Fleisches bewirkt. Ein Fachmann hat beispielsweise den Mikroorganismus Schimmelpilz Thamnidium elegans bisher nur zur Beschleunigung der Fleischreifung verwendet, bis maximal 10 Tagen. Bisher konnte der Fachmann jedoch keinen Nutzen erkennen Mikroorganismen wie Schimmelpilze für die Fleischreifung von Frischfleisch während mehr als 10 Tagen anzuwenden. Der Fachmann wurde auf Grunde seines Fachwissens sogar davon abgehalten eine längere Fleischreifung mit Mikroorganismen wie z.B. den Schimmelpilz Thamnidium elegans in Betracht zu ziehen, da ihm mehrere Probleme bekannt sind, die sich bei längerer Lagerdauer ergeben. Eine Verlängerung der Fleischreifung über 10 Tage hinaus birgt ein erhebliches Risiko, dass das Fleisch verdirbt, insbesondere da Bakterien sich in Funktion der Zeit exponentiell vermehren, sodass mit jedem zusätzlichen Tag ein erhebliches Risiko besteht, dass das Fleisch verdirbt. Ein Fachmann hätte daher die aussergewöhnlich lange Dauer der Fleischreifung von zumindest 21 Tagen und mehr nicht in Betracht gezogen. Eine Fleischreifung von mehr als 10 Tagen führt zudem zu Wasserverlust sowie zu einer angetrockneten Oberfläche beziehungsweise zu einer Krustenbildung an der Fleischoberfläche. Diese Kruste kann nicht mehr verwendet werden und bildet somit einen Fleischverlust. Der Wasserverlust bewirkt zudem einen Gewichtsverlust. Um diese Verluste zu vermeiden hat ein Fachmann auch aus wirtschaftlichen Gründen eine Fleischreifung von mehr als 10 Tagen nicht in Betracht gezogen.

Eine Fleischreifung von zumindest 21 Tagen unter Verwendung der erfindungsgemässen Mikroorganismen hat zur Folge, dass das Fleisch vom Mikroorganismus zumindest teilweise durchwachsen beziehungsweise durchdrungen wird. Dies bewirkt ein besonders zartes, mit einer angenehmen bis intensiven Geschmacksnote versehenes Fleisch.

Bei der erfindungsgemässen Fleischreifung gilt es sicherzustellen, dass das Fleisch nicht verdirbt. Unter üblichen Bedingungen weisen die Bakterien ein schnelleres Wachstum auf als die erfindungsgemässen Mikroorganismen. Daher ist es erforderlich die Verfahrensparameter der Fleischreifung und den Einsatz der erfindungsgemässen Mikroorganismen so zu wählen, dass das Wachstum der Bakterien behindert und das Wachstum des Schimmelpilzes gefördert wird. In einem besonders vorteilhaften Verfahren wird als Mikroorganismus ein Schimmelpilz verwendet und mit einer Sporendichte von zumindest $10^9$ Sporen pro $cm^2$ auf die Oberfläche des Fleisches aufgetragen. Dieser Auftrag erfolgt vorzugsweise innerhalb der ersten zwei bis vier Tage nach der Schlachtung des Tiers. Die antibakterielle Wirkung des Schimmelpilzes behindert in dieser hohen Konzentration das Wachstum der Bakterien besonders gut, wobei die Temperatur und die Luftfeuchtigkeit so angewendet werden, dass diese das Wachstum des Schimmelpilzes fördern und das Wachstum bzw. die Vermehrung der Bakterien behindern. Dadurch ist sichergestellt, dass das Fleisch auch bei Lagerzeiten zwischen 21 bzw. 28 und 100 Tagen nicht verdirbt.

Der Schimmelpilz wird vorteilhafterweise in flüssiger Form als Zellsuspension des Pilzes möglichst flächendeckend auf die Oberfläche des Fleisches aufgetragen, mit einer Sporendichte von vorzugsweise $10^9$ Sporen pro $cm^2$ bis $10^{15}$ Sporen pro $cm^2$, vorteilhafterweise mit $10^{12}$ Sporen pro $cm^2$ bis $10^{15}$ Sporen pro $cm^2$ oder auch mit $10^9$ Sporen pro $cm^2$ bis $10^{12}$ Sporen pro $cm^2$. Besonders vorteilhaft ist ein gleichmässiger Auftrag, um ein Wachstum der Bakterien an der gesamten Oberfläche des Fleisches zu behindern bzw. zu verhindern. Die Sporen des Schimmelpilzes könnten jedoch auch z.B. mit Hilfe von Druckluft auf die Oberfläche des Fleisches gesprüht werden.

Wird als Mikroorganismus ein Schimmelpilz verwendet, so ist das Fleisch nach Ablauf der Lagerzeit von zumindest 21 Tagen zumindest teilweise, und vorzugsweise vollständig vom Schimmelpilz durchwachsen, und weist an der Oberfläche eine Kruste auf, welche vollständig vom Schimmelpilz bedeckt ist. Nach dem Ablauf der Lagerzeit wird diese Kruste abgeschnitten und entsorgt, und das Fleisch ist somit verkaufsfertig. Überraschenderweise hat sich gezeigt, dass das erfindungsgemässe Verfahren trotz des entstehenden Fleisch- und Gewichtsverlustes wirtschaftlich vorteilhaft ist, da ein Konsument bereit ist für die aussergewöhnliche Qualität des mit dem erfindungsgemässen Verfahren veredelten Fleisches einen bedeutenden Mehrpreis zu bezahlen.

**Kurze Beschreibung der Figuren**

Die zur Erläuterung verwendeten Figuren zeigen:

Fig. 1 Eine international anerkannte Klassierung von Fleisch bezüglich Fleischigkeit und Fettgehalt;

Fig. 2 eine Unterteilung der Fleischigkeitsklassen;

Fig. 3 eine Unterteilung der Fettgewebeklassen;

Fig. 4 eine Grafik mit der Entwicklung der Festigkeit und des Geschmacks in Funktion der Zeit für unterschiedliche Mikroorganismen;

Fig. 5 eine Grafik mit der Entwicklung der Festigkeit und des Geschmacks bei einer schnelleren Endreife.

**Wege zur Ausführung der Erfindung**

Beim erfindungsgemässen Verfahren zur Fleischreifung wird das Frischfleisch am Knochen gereift. In einem vorteilhaften Verfahren wird das Frischfleisch offen in einem Kühlraum gelagert und am Knochen gereift. Das Frischfleisch ist dabei während der Fleischreifung nicht verpackt, insbesondere auch nicht unter Vakuum verpackt. In einem weiteren vorteilhaften Verfahren zur Fleischreifung wird das Fleisch am Knochen gereift und ist dabei im Kühlraum in Behältern oder Beuteln verpackt, wobei diese nicht unter Vakuum stehen sodass zur Fleischreifung Luft verfügbar ist.

Bei den erfindungsgemässen Verfahren wird während der Fleischreifung keinerlei Antibiotika verwendet.

Der Knochen wird vorteilhafterweise am Frischfleisch belassen, könnte jedoch für gewisse Fleischstücke auch abgelöst werden. Um ein besonders vorteilhaftes Aroma zu erlangen ist es vorteilhaft den Knochen am Frischfleisch zu belassen. Zudem wird das Fleisch mit dem Mikroorganismus in Kontakt gebracht. Das Verfahren wird nachfolgend am Beispiel der Verwendung des Schimmelpilzes *Thamnidium elegans* und des Schimmelpilzes *Penicillium nalgiovense* im Detail beschrieben. Das erfindungsgemässen Verfahren zur Fleischreifung umfassend eine Behandlung mit dem genannten Schimmelpilz fördert den Geschmack und/oder erhöht die Zartheit und/oder die Saftigkeit des Fleisches. Unter dem Begriff Schimmelpilz *"Thamnidium elegans"* werden all dessen natürliche Stämme verstanden.

In einem beispielhaften Verfahren wird der Schimmelpilz zu Beginn der zweiten Phase der Fleischreifung, das heisst nach der vollständig abgeschlossenen Totenstarre des Muskels (1-2 Tage) mit dem Fleisch (Kalb, Rind, Lamm, Pferd, Schwein usw.) in Kontakt gebracht. Dabei werden die Sporen des Schimmelpilzes *Thamnidium elegans* beziehungsweise des Schimmelpilzes *Penicillium nalgiovense* beispielsweise durch Aufstreichen aufgebracht oder durch Eintauchen des Fleisches. *Tamnidium elegans* durchwächst das Fleisch während der nachfolgenden Lagerung zumindest teilweise und unterstützt mit verschiedenen Proteasen den Abbau der Totenstarre und des Kollagens. Dies führt zu einer Verbesserung der Zartheit. Dabei ist es nicht erforderlich, dass der Schimmelpilz das Fleisch vollständig durchwächst. Es genügt auch ein teilweises Durchwachsen. Es hat sich gezeigt, dass die vom Schimmelpilz erzeugten Enzyme, Proteasen und Lipasen, bis ins Innere des Fleisches diffundieren und dabei die vorhin beschriebene Wirkung erzeugen. Es ist daher nicht erforderlich, dass der Schimmelpilz sich selbst am Ort befinden muss um die Wirkung zu erzielen. Ausgehend vom Ort, an welchem die Enzyme vom Schimmelpilz produziert und sekretiert werden, diffundieren die Enzyme weiter durch das Fleisch, wobei dies vorzugsweise homogen im Fleisch diffundieren. Zusätzlich hat *Thamnidium elegans* eine nussige Geschmacksnote im Fleisch zur Folge, welche von Fachleuten sehr geschätzt wird und als eine Eigenschaft für ein optimal gereiftes Fleisch gilt. Würde ein anderer Mikroorganismus verwendet, beispielsweise der Schimmelpilz *Penicillium nalgiovense,* so hätte dies eine nach Salami erinnernde Geschmacksnote im Fleisch zur Folge.

Ein Beimpfen des Fleisches mit *Thamnidium elegans* oder mit *Penicillium nalgiovense* erfolgt beispielsweise wie nachfolgend beschrieben:

Nach erfolgter Kultivierung des Schimmelpilzes kann die Kultur für die Beimpfung des Fleisches verwendet werden. Die eigentliche Beimpfung findet im Kühlraum gleich zu Beginn der Fleischlagerung, das heisst vorzugsweise 1 bis 3 Tage nach der Schlachtung des Tieres statt. Dabei werden die Pilzsporen und -hyphen zum Bespiel mit einem handelsüblichen Pinsel von der Festmediumkultur abgetragen und auf das Fleisch appliziert bzw. aufgetragen. Die auf die Oberfläche des Fleisches aufgebrachte Sporendichte beträgt etwa $10^9$ Sporen pro $cm^2$ bis $10^{15}$ Sporen pro $cm^2$. Dabei ist darauf zu achten, dass dies gleichmässig und flächendeckend geschieht. Dieser Vorgang kann falls erforderlich während der ersten Woche im Abstand von 2 und 4 Tagen wiederholt werden. Es kann sich auch als vorteilhaft erweisen das ganze Fleischstück kurz in der Flüssigkeit einzutauchen.

Auch in einem Kühlraum mit einer hohen Anzahl an bereits mit Schimmelpilz bewachsenen Fleischstücken reicht es nicht das Frischfleisch über die in der Luft zirkulierenden Pilzsporen zu beimpfen. Auch in diesem Fall ist beispielsweise das vorhin beschriebene Verfahren oder beispielsweise ein Besprühen erforderlich, um eine genügen hohe Sporendichte auf der Fleischoberfläche zu gewährleisten.

Vorzugsweise wird während der Fleischreifung eine hohe relative Luftfeuchtigkeit verwendet. Ist die Luftfeuchtigkeit zu niedrig trocknet das Fleisch zu stark aus. Bezüglich der Luftfeuchtigkeit gilt es jedoch zu beachten, dass diese auch nicht zu hoch ist, da sich sonst Wasser auf dem Fleisch niederschlagen könnte, sodass auf dem Fleisch eine Schleimschicht entstehen könnte, was den Nachteil ergäbe, dass diese Schleimschicht ein Nährboden für unerwünschte Bakterien darstellt. Vorzugsweise wird zur Fleischreifung eine relative Luftfeuchtigkeit im Bereich zwischen 50% und 98% verwendet, insbesondere im Bereich von 75% bis 85 %, am Bevorzugtesten jedoch im Bereich von 85% bis 98%.

Die Lagerung findet bei einer Temperatur von mindestens 0°C statt, damit *Thamnidium elegans* ein genügendes Wachstum aufweist. Im Vergleich dazu weist der Schimmelpilz Penicillium nalgiovense ein leicht unterschiedliches Wachstum in Abhängigkeit der Temperatur auf. Bei einer Verwendung des Schimmelpilzes Penicillium nalgiovense würde die Lagerung bei einer Temperatur von zumindest 1°C erfolgen. Eine Erhöhung der Temperatur hat zur Folge, dass die Gefahr einer Kontamination des Fleisches durch nicht erwünschte Mikroorganismen steigt. Der vorteilhafte Temperaturbereich für die Lagerung des Fleisches mit *Thamnidium elegans* liegt im Bereich zwischen 0°C bis 8 °C und vorzugsweise im Bereich zwischen 2 bis 4°C. Der vorteilhafte Temperaturbereich für die Lagerung des Fleisches mit *Penicillium nalgiovense,* liegt im Bereich zwischen 1°C bis 8 °C und vorzugsweise im Bereich zwischen 4 bis 5°C.

Ein weiterer wichtiger Verfahrensparameter stellt die Zeitdauer der Lagerung dar. Die Zeitdauer wird so gewählt, dass die Totenstarre so weit wie möglich gelöst wird und *Thamnidium elegans oder Penicillium nalgiovense,* derart durch das Fleisch wachsen kann, dass vorteilhafterweise eine homogene Geschmacksverteilung wie auch einen gleichmässigen Kollagenabbau im Fleisch zu gewährleistet ist. Vorzugsweise liegt die Zeitdauer der Lagerung in einem Bereich zwischen 21 und 100 Tagen, und insbesondere in einem Bereich zwischen 21 bzw. 28 und 50 Tagen, insbesondere bei etwa 35 Tagen.

Abhängig vom verwendeten Organismus wird das Fleisch besonders vorteilhaft in einem Temperaturbereich zwischen 1 bis 5 °C, bei einer relativen Luftfeuchtigkeit zwischen 75% und 95%, und zwischen 25 bis 45 Tagen gelagert. Noch vorteilhafter wird das Fleisch bei einer Temperatur von etwa 3 °C, bei einer relativen Luftfeuchtigkeit von etwa 85%, und während etwa 35 Tagen gelagert. Das Fleisch wird besonders vorteilhaft bei einer Temperatur zwischen 2 bis 4 °C, bei einer relativen Luftfeuchtigkeit zwischen 85% und 98%, vorzugsweise zwischen 90% und 98%, und zwischen 28 bis 45 Tagen gelagert.

Das Verfahren ist insbesondere zur Behandlung von Rindfleisch geeignet. Das Fleisch aller Rinderrassen ist geeignet, insbesondere das Fleisch Angus, Braunvieh, Simmenthaler, Charolais, Hereford, Wagyu oder Bison. Das Verfahren ist jedoch auch zur Behandlung von Kalb-, Lamm-, Pferde- oder Schweinefleisch geeignet.

Damit das Fleisch während der langen Lagerzeit nicht austrocknet wird vorteilhafterweise ein Fleisch mit einem relativ hohen Fettanteil verwendet, denn das Fett hat die Fähigkeit Wasser zu binden. Zudem wird vorteilhafterweise ein Fleisch mit einer hohen Fleischigkeit verwendet.

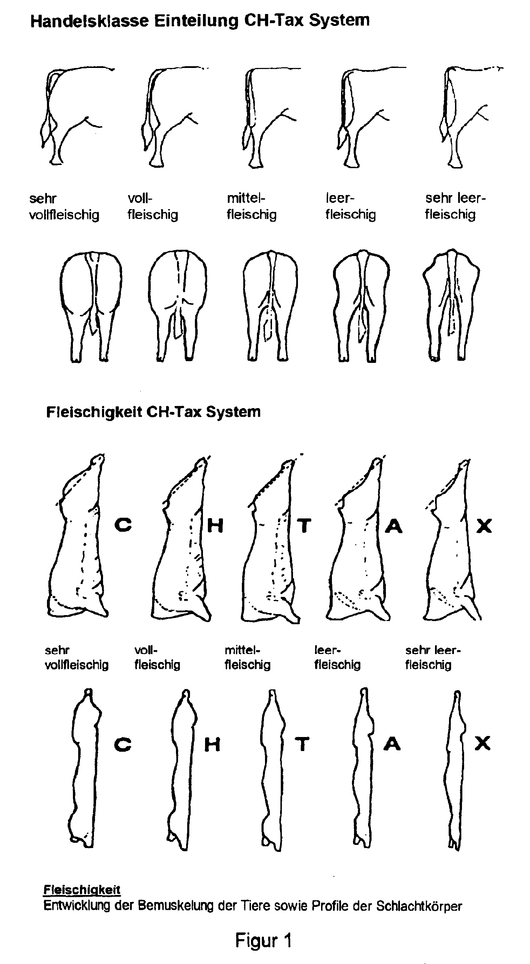

Die Eigenschaften von Schlachtfleisch werden basierend auf einer international anerkannten Klassierung bezüglich Fleischigkeit und Fettgehalt klassiert. In der Schweiz wird diese Klassierung als CH-Tax-System bezeichnet, welches Fleisch wie in den Figuren 1 bis 3 dargestellt klassiert.

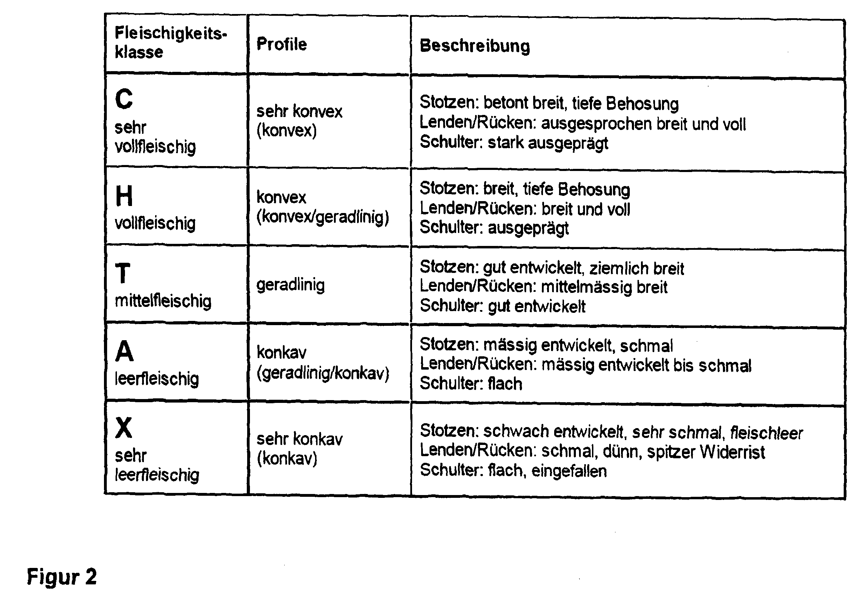

Das verwendete Fleisch für das erfindungsgemässe Verfahren kann, wie in den Figuren 1 und 2 dargestellt, eine Fleischigkeit zwischen C (sehr vollfleischig) und X (sehr leerfleischig) aufweisen. Vorzugsweise wird Fleisch mit einer Fleischigkeit zwischen C (sehr vollfleischig) und T (mittelfleischig) verwendet.

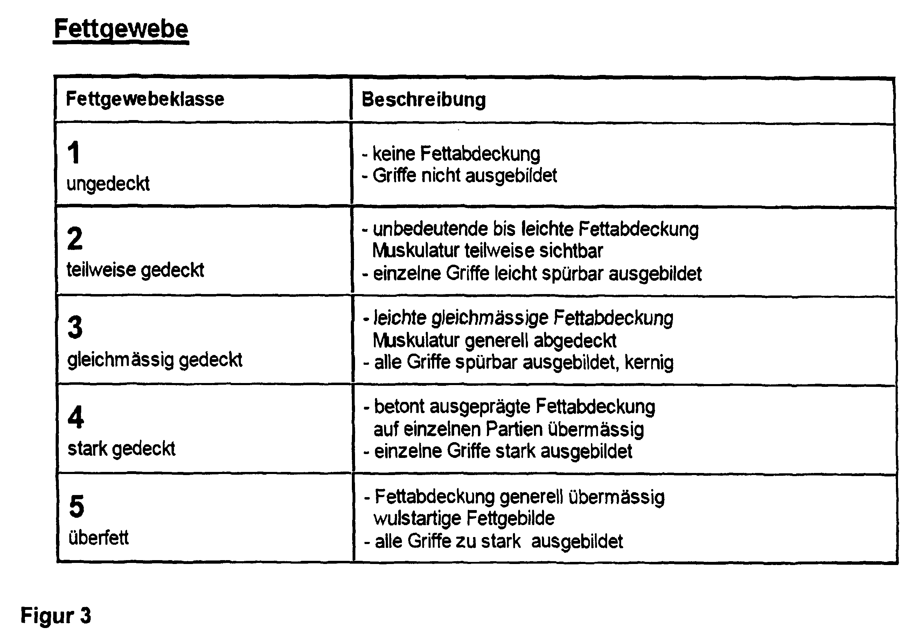

Bezüglich der in Figur 3 dargestellten Fettgewebeklasse wird vorzugsweise ein Fleisch zwischen Klasse 3 (gleichmässig gedeckt) und Klasse 5 (überfett) verwendet. Die Beurteilung des Fettgewebes des Schlachtkörpers (aussagekräftig ist zum Beispiel die Dicke der Fettschicht auf der Innenseite der Brusthöhle) ermöglicht indirekte Aussagen über Ausmästungsgrad, Fleisch-/Fettverhältnisse und Marmorierung des Fleisches.

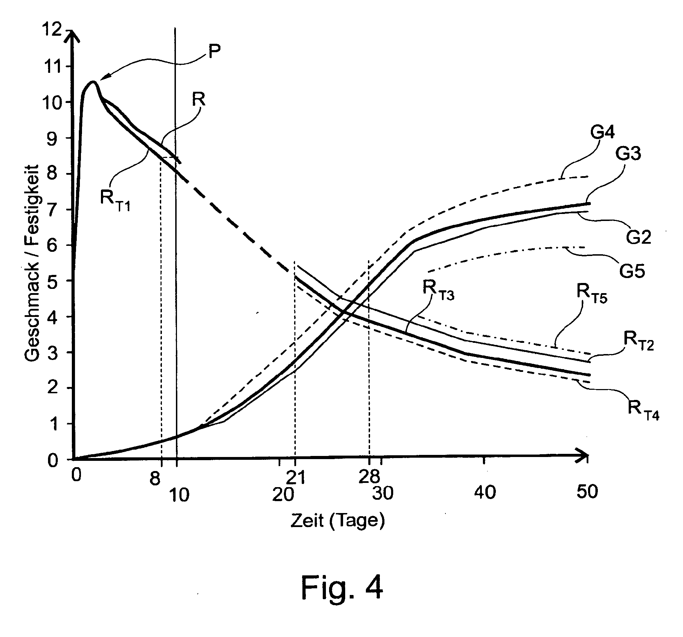

Zur Beurteilung der Fleischqualität kann die Klassierung bezüglich Fleischigkeit und Fettgehalt kombiniert werden. Vorzugsweise wird Fleisch zwischen T3 (Fleischigkeitsklasse T und Fettgewebeklasse 3) und C5 (Fleischigkeitsklasse C und Fettgewebeklasse 5) verwendet. Figur 4 zeigt an Verfahrensbeispielen die Entwicklung der Festigkeit von Rindfleisch sowie die Entwicklung des Geschmacks des Fleisches in Funktion der Zeit schematisch für unterschiedliche Organismen. Die Ordinate zeigt eine Skala von 0 bis 12 und die Abszisse eine Zeitdauer von 50 Tage. Die Festigkeit des Fleisches wurde subjektiv und objektiv getestet und das Ergebnis auf Werte zwischen 0 und 12 skaliert, wobei die Skala so gewählt ist, dass das Fleisch bei 0 am zartesten ist und bei 12 am härtesten ist. Der Verlauf der Festigkeit des Fleisches in Funktion der Zeit bei heute üblicher Reifung, das heisst bei Reifung in einem Vakuumbeutel, und bei einer Reifedauer von maximal 10 Tagen ist in der Reifekurve R dargestellt. Die Festigkeit des Fleisches steigt unmittelbar nach der Schlachtung stark an und erreicht etwa 24 Stunden nach der Schlachtung, nach Abschluss der Totenstarre, im Punkt P die maximale Festigkeit. Danach reduziert sich die Festigkeit des Fleisches durch zelleigenen Enzyme und Mikroorganismen. Nach einer Reifezeit T von maximal 10 Tagen ist das Fleisch ausgereift und steht für den Verkauf bereit. Die Reifekurve $R_{T1}$ zeigt den Verlauf der Festigkeit des Fleisches in Funktion der Zeit beim bekannten "fast aging"-Verfahrens unter Verwendung des Schimmelpilzes Thamnidium elegans. Der Reifekurve $R_{T1}$ ist zu entnehmen, dass das Fleisch bereits nach etwa 8 Tagen dieselbe Festigkeit aufweist wie die Reifekurve R nach 10 Tagen. Die Verwendung des Schimmelpilzes Thamnidium elegans ermöglicht somit eine schnellere Fleischreifung, im dargestellten Beispiel um zwei Tage. Diese Verkürzung der Fleischreifung um zwei Tage ist, unter Berücksichtigung, dass sich Bakterien exponentiell vermehren, von entscheidender Bedeutung, da die Fleischreifung dadurch auch bei höheren Temperaturen durchführbar ist.

Der Reifekurve $R_{T2}$ zeigt den Verlauf der Festigkeit des Fleisches im Bereich der Lagerzeit von mehr als 21 Tagen unter Verwendung des Schimmelpilzes Penicillium nalgiovense. Der Reifekurve $R_{T3}$ zeigt den Verlauf der Festigkeit des Fleisches im Bereich der Lagerzeit von mehr als 21 Tagen unter Verwendung des Schimmelpilzes Thamnidium elegans. Nach 21 Tagen nimmt die Festigkeit des Fleisches immer weniger ab.

Figur 4 zeigt im Geschmacksverlauf G zudem den Geschmack des Fleisches in Funktion der Zeit, wobei der Geschmack in Kurve G2 vorwiegend oder ausschliesslich verursacht wird durch den Schimmelpilz Penicillium nalgiovense und in Kurve G3 durch den Schimmelpilz Thamnidium elegans. Der Geschmack des Fleisches wurde subjektiv mit Versuchspersonen getestet und das Ergebnis auf Werte zwischen 0 und 12 skaliert, wobei die Skala so gewählt ist, dass das Fleisch bei 12 am Besten schmecken würde. Die Geschmacksverläufe G2, G3 in Funktion der Zeit zeigen, dass sich diese innerhalb der ersten 8 bis 10 Tage nur geringfügig entwickeln. Die Geschmacksverläufe G2, G3 zeigt zudem, dass sich der Geschmack nach etwa 40 bis 50 Tagen nicht mehr erhöht, und somit in eine Sättigung gelangt. Die Geschmacksverläufe G2, G3 zeigen zudem, dass sich bei den zur Fleischreifung verwendeten Temperatur und Luftfeuchtigkeit der Geschmacksverlauf G2 des Schimmelpilzes Penicillium nalgiovense bezüglich des Geschmacksverlaufs G3 des Schimmelpilzes Thamnidium elegans um etwa 5 Tage verzögert entwickelt. Wie aus den Reifekurve $R_{T2}$ des Schimmelpilzes Penicillium nalgiovense und der Reifekurve $R_{T3}$ des Schimmelpilzes Thamnidium elegans ersichtlich, verläuft die Reifekurve $R_{T2}$ im Vergleich Reifekurve $R_{T3}$ um etwa 5 Tage verzögert. Abhängig vom verwendeten Organismus und natürlich auch von der verwendeten Temperatur und Luftfeuchtigkeit, verläuft die Geschmacksentwicklung beziehungsweise die Reifung in Funktion der Zeit unterschiedlich, insbesondere verzögert beziehungsweise beschleunigt.

Aus der in Figur 4 dargestellten Grafik ist ersichtlich, dass ein Schimmelpilz wie Penicillium nalgiovense oder Thamnidium elegans nach 10 Tagen nur eine Randschicht des Fleisches durchwachsen hat, wogegen bei Lagerung von 21 und mehr Tagen, je nach Fleischstück, das Fleisch bereits zu einem grossen Teil vom Schimmelpilz durchwachsen ist und die biochemische Aktivität der Pilzenzyme auch im Fleischinneren wirken können. Bei einer Lagerzeit von zumindest 21 Tagen ist der maximal mögliche Geschmack etwa zur Hälfte entwickelt. Besonders vorteilhaft ist eine Lagerung zwischen 25 bis 45 Tagen beziehungsweise eine Lagerung zwischen 28 und 50 Tagen. Wie aus dem Geschmacksverlauf G2 bzw. G3 ersichtlich weist dieser nach etwa 25 bis 28 Tagen eine abnehmende Steigung auf, und die Kurve des Geschmacksverlaufes G2 bzw. G3 flacht danach zunehmend ab. Zudem nimmt die Zartheit nach 25 bis 28 Tagen nur noch beschränkt zu, sodass es aus Kosten-Nutzen-Überlegungen vorteilhaft ist das erfindungsgemässe Reifeverfahren nach etwa 25 bis 28 Tagen zu beenden. Beim erfindungsgemässen Verfahren wird die Festigkeit des Fleisches über mindestens 21 Tage und vorzugsweise über zumindest 25 oder 28 Tage stetig reduziert, beziehungsweise die Zartheit des Fleisches stetig gesteigert. Zudem wird der durch den Schimmelpilz Penicillium nalgiovense bzw. Thamnidium elegans hervorgerufene Geschmack erheblich gesteigert, sodass nach 21 bis 28 Tagen ein aussergewöhnlich zartes, stark aromatisches, Fleisch vorliegt. Eine Reifezeit von mehr als 21 bzw. 28 Tagen bewirkt eine zusätzlich Erhöhung der Zartheit des Fleisches sowie des Geschmacks, wobei sich die Zunahme, wie aus Figur 4 ersichtlich, in Funktion der Zeit verringert, und bezüglich Geschmack nach etwa 50 Tagen kaum mehr eine Zunahme stattfinden, und bezüglich Zartheit nach etwa 100 Tagen kaum mehr eine Zunahme stattfindet. Figur 4 zeigt die Entwicklung des Geschmacksverlaufs G2 bzw. G3 sowie die Reifekurve $R_{T2}$, $R_{T3}$ in Funktion der Zeit beispielhaft an Hand der Schimmelpilze Penicillium nalgiovense und Thamnidium elegans. Der Verlauf des Geschmacksverlaufes G2 bzw. G3 sowie der Reifekurve $R_{T2}$, $R_{T3}$ ist abhängig vom verwendeten Mikroorganismus, sowie der Temperatur und der Luftfeuchtigkeit. Figur 4 zeigt mit den teilweise nur Abschnittweise dargestellten Verläufen G4 und G5 beispielhaft den Geschmacksverlauf und mit den Reifekurven $R_{T4}$, $R_{T5}$ beispielhaft den Verlauf der Festigkeit bei Verwendung anderer Mikroorganismen, wobei auch diese Verläufe zudem von Temperatur- und Luftfeuchtigkeitsparameter abhängig sind. In Kenntnis der erfindungsgemässen Idee können die Temperatur- und Luftfeuchtigkeitsparameter im beanspruchten Temperaturbeziehungsweise Luftfeuchtigkeitsbereich auf einfache Weise abhängig vom verwendeten Mikroorganismus optimiert werden, um einen entsprechenden Geschmacksverlauf beziehungsweise eine gewünschte Reifekurve zu erlangen.

Für ein besonders zartes und geschmacksvolles Fleisch erfolgt der Reifeprozess bei einer Temperatur zwischen 2 bis 4 °C, sowie bei einer relativen Luftfeuchtigkeit zwischen 85% und 98%, vorzugsweise zwischen 90% und 98%, um dadurch ein Wachstum von Mikroorganismen wie Bakterien zu behindern und um gleichzeitig das Wachstum des Schimmelpilzes Thamnidium elegans bzw. Penicillium nalgiovense zu fördern. Unter diese Bedingungen wird das Fleisch vorzugsweise zwischen 28 bis 45 Tagen gelagert, um die in Figur 4 dargestellte Zartheit und den Geschmack zu erlangen.

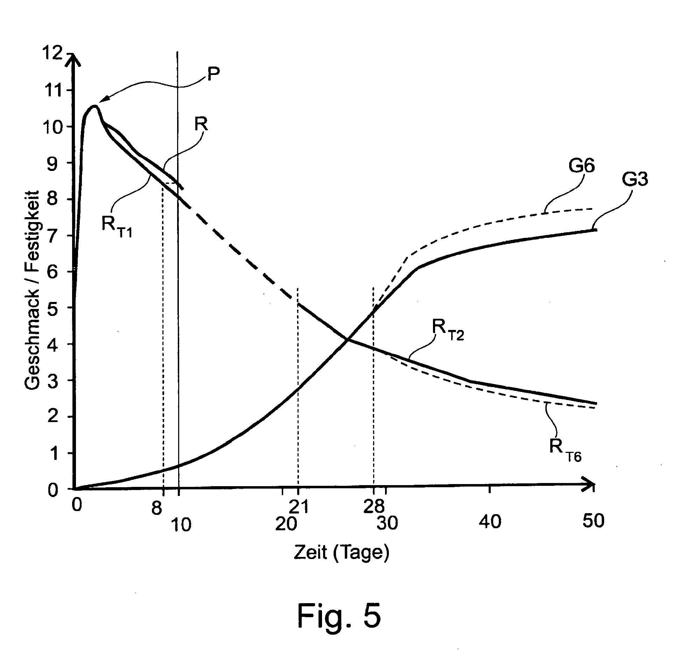

Die Geschwindigkeit der Fleischreifung kann über die Temperatur beeinflusst werden. Figur 5 zeigt ein Verfahrensbeispiel zur Erlangung einer schnelleren Endreifung des Fleisches. Figur 5 zeigt die Fleischreifung beispielhaft unter Verwendung des Schimmelpilzes Thamnidium elegans, wobei dieser Effekt auch mit anderen vorhin genannten Mikroorganismen erzielbar ist. Die Kurve G3 zeigt wie in Figur 4 den Verlauf des Geschmacks des Rindfleisches in Funktion der Zeit. Die Reifekurve RT2 zeigt den Verlauf der Festigkeit in Funktion der Zeit. Dabei wird das Rindfleisch bei einer Temperatur zwischen 2 bis 4°C gelagert. Im Verfahrensbeispiel gemäss Figur 5 wird nun die Temperatur nach 28 Tagen auf 6°C erhöht und das Rindfleisch dadurch anschliessend bei 6°C gelagert. Dieser Temperaturanstieg an eine schnellere Endreifung zur Folgen, indem, wie im Verlauf G6 dargestellt, der Geschmacksverlauf stärker zunimmt. Der Temperaturanstieg hat auch einen Einfluss auf die Reifekurve, wie dies im Verlauf der Reifekurve RT6 dargestellt ist. Ein Vorteil dieser Temperaturerhöhung liegt darin, dass die Gesamtlagerdauer, die erforderlich ist um ein besonders zartes und geschmacksvolles Fleisch zu erlagen, verkürzt werden kann. Diese schnellere Endreifung ist insbesondere deshalb möglich, weil der während 28 Tagen gewachsene Schimmelpilz das Wachstum von Bakterien behindert, sodass eine Temperaturerhöhung nach 28 Tagen möglich ist, ohne dass die Gefahr besteht, dass vorhandene Bakterien das Fleisch verderben könnten. Das in Figur 5 dargestellte Ausführungsbeispiel wurde an Hand eines Beispiels gezeigt bei welchem die Temperatur nach 28 Tagen erhöht wurde. Die Temperatur könnte jedoch auch früher, d.h. vor 28 Tagen, oder auch später erhöht werden. Zur schnelleren Endreifung wird die Temperatur nach einer Lagerdauer von zumindest 21 Tagen erhöht, wobei die Temperatur vorzugsweise um 1 bis 2 °C erhöht wird.

**Patentansprüche**

1. Verfahren zur Veredelung von Frischfleisch ohne zusätzliche Salzzugabe, wobei zur Reifung des Fleisches zumindest ein Mikroorganismus aus der Gruppe:

     Penicillium nalgiovense, Penicillium chysogenum, Penicillium gladioli, Penicillium camemberti, Thamnidium elegans zugegeben wird, wobei das Fleisch in einem Temperaturbereich zwischen 0 bis 8 °C gelagert wird, wobei das Fleisch bei einer relativen Luftfeuchtigkeit zwischen 50% und 98% gelagert wird, und wobei das Fleisch während einer Zeitdauer zwischen 21 bis 100 Tagen gelagert wird.

2. Verfahren nach Anspruch 1, **dadurch gekennzeichnet, dass** zumindest einer der Parameter Temperatur, Luftfeuchtigkeit und Zeitdauer derart beeinflusst wird, dass das Fleisch während der Reifung immer einen $a_w$-Wert von grösser oder gleich 0.96 aufweist.

3. Verfahren nach Anspruch 1, **dadurch gekennzeichnet, dass** das Fleisch zwischen 28 bis 100 Tagen gelagert wird.

4. Verfahren nach einem der vorhergehenden Ansprüche, **dadurch gekennzeichnet, dass** der Schimmelpilz mit einer Sporendichte von zumindest $10^9$ Sporen pro $cm^2$ auf die Oberfläche des Fleisches aufgetragen wird, vorzugsweise mit einer Sporendicht zwischen $10^{12}$ Sporen pro $cm^2$ und $10^{15}$ Sporen pro $cm^2$.

5. Verfahren nach einem der vorhergehenden Ansprüche, **dadurch gekennzeichnet, dass** das Fleisch in einem Temperaturbereich zwischen 1 bis 7 °C gelagert wird, und vorzugsweise in einem Temperaturbereich zwischen 3 bis 7 °C.

6. Verfahren nach einem der vorhergehenden Ansprüche, **dadurch gekennzeichnet, dass** das Fleisch bei einer relativen Luftfeuchtigkeit zwischen 75% und 98% gelagert wird.

7. Verfahren nach einem der vorhergehenden Ansprüche, **dadurch gekennzeichnet, dass** das Fleisch in einem Temperaturbereich zwischen 1 bis 7 °C, bei einer relativen Luftfeuchtigkeit zwischen 75% und 98%, und zwischen 21 bis 70 Tagen gelagert wird.

8. Verfahren nach einem der vorhergehenden Ansprüche, **dadurch gekennzeichnet, dass** die Temperatur nach einer Lagerdauer von zumindest 21 Tagen erhöht wird, um eine schnellere Endreifung zu bewirken.

9. Verfahren nach einem der vorhergehenden Ansprüche, **dadurch gekennzeichnet, dass** als Fleisch Kalb-, Rind-, Lamm-, Pferde- oder Schweinefleisch verwendet wird.

10. Verwendung zumindest eines Mikroorganismus aus der Gruppe:

Penicillium nalgiovense, Penicillium chysogenum, Penicillium gladioli, Penicillium camemberti, Thamnidium elegans zur Reifung von Fleisch ohne zusätzliche Salzzugabe, in einem Temperaturbereich zwischen 0 bis 8 °C, bei einer Luftfeuchtigkeit zwischen 50 bis 98% und während einer Zeitdauer von 21 bis 100 Tagen.

**Claims**

1. Method for refining fresh meat without additional addition of salt, wherein at least one microorganism from the group consisting of:

Penicillium nalgiovense, Penicillium chrysogenum, Penicillium gladioli, Penicillium camemberti, Thamnidium elegans is added for ageing of the meat, wherein the meat is stored in a temperature range between 0 and 8°C, wherein the meat is stored at a relative humidity of air between 50% and 98%, and wherein the meat is stored for a time period between 21 and 100 days.

2. Method according to Claim 1, **characterized in that** at least one of the parameters temperature, humidity of air and time period is influenced in such a manner that the meat during ageing always has an $a_w$ value of greater than or equal to 0.96.

3. Method according to Claim 1, **characterized in that** the meat is stored between 28 and 100 days.

4. Method according to any one of the preceding claims, **characterized in that** the mould having a spore density of at least $10^9$ spores per $cm^2$ is applied to the surface of the meat, preferably with a spore density between $10^{12}$ spores per $cm^2$ and $10^{15}$ spores per $cm^2$.

5. Method according to any one of the preceding claims, **characterized in that** the meat is stored in a temperature range between 1 and 7°C, and preferably in a temperature range between 3 and 7°C.

6. Method according to any one of the preceding claims, **characterized in that** the meat is stored at a relative humidity of air between 75% and 98%.

7. Method according to any one of the preceding claims, **characterized in that** the meat is stored in a temperature range between 1 and 7°C, at a relative humidity of air between 75% and 98%, and between 21 and 70 days.

8. Method according to any one of the preceding claims, **characterized in that** the temperature is elevated after a storage period of at least 21 days, in order to effect faster final ageing.

9. Method according to any one of the preceding claims, **characterized in that** veal, beef, lamb, horsemeat or pork is used as the meat.

10. Use of at least one microorganism from the group consisting of:

Penicillium nalgiovense, Penicillium chrysogenum, Penicillium gladioli, Penicillium camemberti, Thamnidium elegans for ageing meat without additional addition of salt, in a temperature range between 0°C and 8°C, at a humidity of air between 50 and 98%, and for a time period from 21 to 100 days.

**Revendications**

1. Procédé pour l'affinage de viande fraîche sans addition supplémentaire de sel, dans lequel on ajoute pour la maturation de la viande au moins un micro-organisme choisi dans le groupe : Penicillium nalgiovense, Pénicillium chrysogenum, Penicillium gladioli, Pénicillium camemberti, Thamnidium elegans, dans lequel la viande est entreposée dans une plage de température comprise entre 0 °C et 8 °C, dans lequel la viande est entreposée à une humidité relative de l'air comprise entre 50 % et 98 %, et dans lequel la viande est entreposée pendant une durée comprise entre 21 et 100 jours.

2. Procédé selon la revendication 1, **caractérisé en ce qu'**au moins l'un des paramètres température, humidité de l'air et durée est influencé par le fait que pendant la maturation la viande présente toujours une valeur $a_w$ supérieure ou égale à 0,96.

3. Procédé selon la revendication 1, **caractérisé en ce que** la viande est entreposée pendant une durée comprise entre 28 et 100 jours.

4. Procédé selon l'une quelconque des revendications précédentes, **caractérisé en ce qu'**on applique à la surface de la viande la moisissure à une densité de spores d'au moins $10^9$ spores par $cm^2$, de préférence à une densité de spores comprise entre $10^{12}$ spores par $cm^2$ et $10^{15}$ spores par $cm^2$.

5. Procédé selon l'une quelconque des revendications précédentes, **caractérisé en ce que** la viande est entreposée dans une plage de température comprise entre 1 et 7 °C, et de préférence dans une plage de température comprise entre 3 et 7 °C.

6. Procédé selon l'une quelconque des revendications précédentes, **caractérisé en ce que** la viande est entreposée à une humidité relative de l'air comprise entre 75 % et 98 %.

7. Procédé selon l'une quelconque des revendications précédentes, **caractérisé en ce que** la viande est entreposée dans une plage de température comprise entre 1 et 7 °C, à une humidité relative de l'air comprise entre 75 % et 98 % et pendant une durée comprise entre 21 et 70 jours.

8. Procédé selon l'une quelconque des revendications précédentes, **caractérisé en ce qu'**après une durée d'entreposage d'au moins 21 jours on élève la température afin de provoquer une maturation finale plus rapide.

9. Procédé selon l'une quelconque des revendications précédentes, **caractérisé en ce qu'**on utilise comme viande de la viande de veau, de boeuf, d'agneau, de cheval ou de porc.

10. Utilisation d'au moins un micro-organisme choisi dans le groupe :

Pénicillium nalgiovense, Pénicillium chrysogenum, Pénicillium gladioli, Pénicillium camemberti, Thamnidium elegans, pour la maturation de viande sans addition supplémentaire de sel, dans une plage de température comprise entre 0 °C et 8 °C, à une humidité relative de l'air comprise entre 50 et 98 % et pendant une durée de 21 à 100 jours.

## Handelsklasse Einteilung CH-Tax System

| sehr vollfleischig | voll- fleischig | mittel- fleischig | leer- fleischig | sehr leer- fleischig |

## Fleischigkeit CH-Tax System

| sehr vollfleischig | voll- fleischig | mittel- fleischig | leer- fleischig | sehr leer- fleischig |

**Fleischigkeit**
Entwicklung der Bemuskelung der Tiere sowie Profile der Schlachtkörper

## Figur 1

| Fleischigkeits-klasse | Profile | Beschreibung |
|---|---|---|
| **C**<br><br>sehr<br>vollfleischig | sehr konvex<br>(konvex) | Stotzen: betont breit, tiefe Behosung<br>Lenden/Rücken: ausgesprochen breit und voll<br>Schulter: stark ausgeprägt |
| **H**<br><br>vollfleischig | konvex<br>(konvex/geradlinig) | Stotzen: breit, tiefe Behosung<br>Lenden/Rücken: breit und voll<br>Schulter: ausgeprägt |
| **T**<br><br>mittelfleischig | geradlinig | Stotzen: gut entwickelt, ziemlich breit<br>Lenden/Rücken: mittelmässig breit<br>Schulter: gut entwickelt |
| **A**<br><br>leerfleischig | konkav<br>(geradlinig/konkav) | Stotzen: mässig entwickelt, schmal<br>Lenden/Rücken: mässig entwickelt bis schmal<br>Schulter: flach |
| **X**<br><br>sehr<br>leerfleischig | sehr konkav<br>(konkav) | Stotzen: schwach entwickelt, sehr schmal, fleischleer<br>Lenden/Rücken: schmal, dünn, spitzer Widerrist<br>Schulter: flach, eingefallen |

**Figur 2**

EP 2 701 538 B1

# Fettgewebe

| Fettgewebeklasse | Beschreibung |
|---|---|
| **1**<br>ungedeckt | - keine Fettabdeckung<br>- Griffe nicht ausgebildet |
| **2**<br>teilweise gedeckt | - unbedeutende bis leichte Fettabdeckung<br>  Muskulatur teilweise sichtbar<br>- einzelne Griffe leicht spürbar ausgebildet |
| **3**<br>gleichmässig gedeckt | - leichte gleichmässige Fettabdeckung<br>  Muskulatur generell abgedeckt<br>- alle Griffe spürbar ausgebildet, kernig |
| **4**<br>stark gedeckt | - betont ausgeprägte Fettabdeckung<br>  auf einzelnen Partien übermässig<br>- einzelne Griffe stark ausgebildet |
| **5**<br>überfett | - Fettabdeckung generell übermässig<br>  wulstartige Fettgebilde<br>- alle Griffe zu stark ausgebildet |

**Figur 3**

EP 2 701 538 B1

Fig. 4

Fig. 5

**IN DER BESCHREIBUNG AUFGEFÜHRTE DOKUMENTE**

**In der Beschreibung aufgeführte Patentdokumente**

- US 3056679 A
- US 3128191 A
- GB 875339 A
- US 2816836 A